(19) Europäisches Patentamt
European Patent Office
Office européen des brevets

(11) Publication number: **0 211 266**
**B1**

(12) **EUROPEAN PATENT SPECIFICATION**

(45) Date of publication of patent specification: **05.12.90**

(51) Int. Cl.$^5$: **A 61 K 31/135**, A 61 K 9/22

(21) Application number: **86109445.6**

(22) Date of filing: **10.07.86**

(54) **Continuous release phenylethanolamine derivative compositions.**

(30) Priority: **29.07.85 US 759702**

(43) Date of publication of application:
**25.02.87 Bulletin 87/09**

(45) Publication of the grant of the patent:
**05.12.90 Bulletin 90/49**

(84) Designated Contracting States:
**AT BE CH DE FR GB IT LI LU NL SE**

(56) References cited:
**EP-A-0 013 949**
**EP-A-0 049 728**
**EP-A-0 080 595**
**US-A-2 517 513**
**US-A-4 552 897**

**The file contains technical information submitted after the application was filed and not included in this specification**

(73) Proprietor: **AMERICAN CYANAMID COMPANY**
**1937 West Main Street P.O. Box 60**
**Stamford Connecticut 06904-0060 (US)**

(72) Inventor: **Fishbein, Richard**
**18 Meadow Run Road**
**Skillman New Jersey 08558 (US)**
Inventor: **Mancini Cady, Susan**
**1501 Revere Road**
**Yardley Pennsylvania 19067 (US)**
Inventor: **Chaudhuri, Ajit Kumar**
**7 North Cadillac Drive**
**Somerville New Jersey 08876 (US)**

(74) Representative: **Wächtershäuser, Günter, Dr.**
**Tal 29**
**D-8000 München 2 (DE)**

**EP 0 211 266 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Background of the Invention

The difficulties encountered in the development of methods and compositions which continuously release pharmaceutical preparations in a uniform manner over extended periods of time are well known.

Recent developments in the area of controlling the release of drugs have been disclosed which describe methods for controlling the release of drugs by microencapsulation and containment within a biodegradable matrix.

Substitution products of 1-(amino-dihalophenyl)-2-aminoethanes, and the acid addition salts thereof are known as useful agents for enhancing the blood circulation, and as bronchodilators, analgesics, sedatives, antipyretics, antiphlogistics and antitusives in warm-blooded animals. The preparation of other related 1-(amino-dihalophenyl)-2-aminoethanols and their derivatives are known in the art. These applications disclose uses selected from analgesics, broncholytic, antiinflammatory, uterine spasmolytic, β-mimetic and/or β-blocking activities, antispasmolytic activity on cross-striped muscle structure, for tocology, reducing blood pressure by peripheral vasodilation and mobilizing body fat, and for treating allergies.

Certain phenylethanolamine derivatives and their use for the depression of fat deposition in warm-blooded animals are disclosed in United States Patent No. 4,407,819. United States Patent No. 4,404,222 discloses certain phenylethanolamine derivatives and their use for enhancing the growth rate of meat-producing animals and improving the efficiency of feed utilization in animals so treated. Recently, Offenlegungsschrift DE 3,306,159 A1 was published describing substituted phenylethylamine derivatives which were said to be growth promoters for pigs, cows, poultry, cats, dogs, rabbits, fur animals, fish, and reptiles.

Summary of the Invention

The present invention provides a novel composition for the parenteral administration of an essentially uniform and continuous amount of a phenylethanolamine derivative over an extended period of time comprising a compacted and partially coated phenylethanolamine derivative, which exhibits some degree of solubility in an aqueous physiological environment. The phenylethanolamine derivative suitable for use in this composition is a Formula I compound, having the structure:

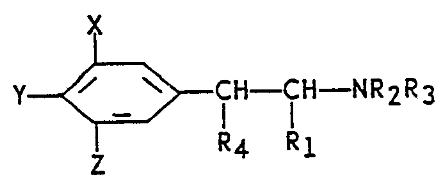

(I)

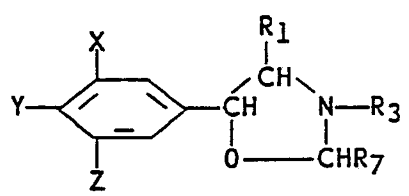

(Ia)

and

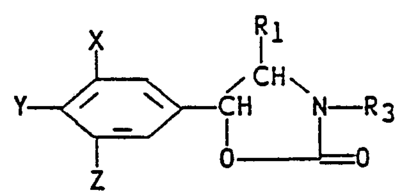

(Ib)

wherein,

X is hydrogen, halogen or —CN;

Y is hydrogen, $NR_8R_9$ or $NHCOR_5$;

Z is hydrogen, halogen, OH, CN, $CF_3$, $COOR_1$, $CONH_2$, $C_1$—$C_4$ alkyl, $C_1$—$C_4$ alkoxy, $NO_2$, $C_1$—$C_4$-dialkylaminomethyl or hydroxymethyl;

$R_1$ is hydrogen or $C_1$—$C_4$ alkyl;

$R_2$ is hydrogen, $C_1$—$C_6$ alkyl, $C_3$—$C_4$ alkenyl, $C_2$—$C_5$ alkanoyl or

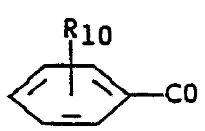 ;

$R_3$ is hydrogen, $C_1$—$C_6$ alkyl, $C_3$—$C_6$ cycloalkyl, methoxypropyl, $C_3$—$C_4$ alkenyl, phenyl, 2-hydroxyethyl, α,α-dimethylphenethyl, benzyl, 3-phenylpropyl or 3-(4-carbomethoxyphenyl)propyl; and when $R_2$ and $R_3$

are taken together with the nitrogen to which they are attached, they represent morpholino or N'-$C_1$—$C_4$ alkylpiperazino;

$R_4$ is hydrogen, OH, $OR_6$ or $SR_{11}$;

$R_5$ is hydrogen, $C_1$—$C_4$ alkyl, $C_1$—$C_4$ alkoxy,

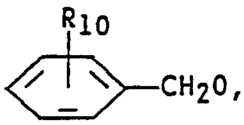, 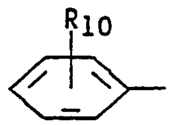 or $N(R_1)_2$ ;

$R_6$ is $C_1$—$C_6$ alkyl, $C_2$—$C_5$ alkanoyl,

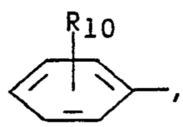, 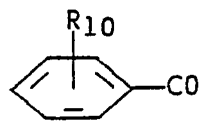 or 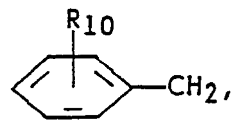, $C_3$–$C_4$ alkenyl;

$R_7$ is hydrogen, $C_1$—$C_4$ alkyl or phenyl;

$R_8$ is hydrogen, $C_1$—$C_4$ alkyl or $C_3$—$C_4$ alkenyl;

$R_9$ is hydrogen, $C_1$—$C_6$ alkyl, $C_4$—$C_6$ cycloalkyl, $C_3$—$C_4$ alkenyl, or benzyl; and when $R_8$ and $R_9$ are taken together with the nitrogen to which they are attached, they represent pyrrolidino;

$R_{10}$ is chloro, dichloro, methyl, dimethyl, methoxy, dimethoxy or nitro; and

$R_{11}$ is $C_1$—$C_6$ alkyl, phenyl or benzyl; and/or is $C_{10}$—$C_{20}$ fatty acid salt.

Surprisingly, it has been found that the partially coated implant of compacted free base Formula (I) compounds and $C_{10}$—$C_{20}$ fatty acid salts thereof show a more constant release of the compound while the uncoated implant of the free base Formula (I) compounds and $C_{10}$—$C_{20}$ fatty acid salts thereof do not show a constant release of the compound.

The invention also is directed to the preparation and administration of said compositions.

Preferred Embodiment of the Invention

The rate of release and dosage of the composition of the invention may be adjusted by the use of the pure Formula (I) compounds or fatty acid salts thereof and coating materials.

A preferred group of compounds for use in this invention have the above Formula (I) structure wherein X is hydrogen or halogen; Y is hydrogen, $NR_8R_9$ or $NHCOR_5$; Z is halogen, OH, CN, $CF_3$, $COOR_1$, $CONH_2$, methyl, methoxy, $NO_2$, $C_1$—$C_4$ dialkylaminomethyl, or hydroxymethyl; and the remaining groups are as hereinbefore defined.

A preferred group of fatty acid salts for use in this invention include the $C_{10}$—$C_{20}$ fatty acid salts of the Formula I compounds.

The composition of the invention may optionally contain up to about 50% by weight of a diluent or mixture of diluents and up to about 5% by weight of a lubricant of mixture of lubricants. Suitable diluents for the invention are ethyl cellulose and castorwax. A suitable lubricant for the invention is magnesium stearate.

The term "aqueous physiological environment" means the body of a warm-blooded animals as well as such an *in vitro* environment which may mimicked by aqueous liquids, such as phosphate buffered solutions at a temperature of 35° to 40°C.

An implant for the administration of phenylethanolamine derivatives may be prepared by admixing a Formula (I) compound in an organic solvent such as methanol, optionally with a fatty acid, if a salt is desired, and then adding the ethyl cellulose and castorwax, The resulting material is isolated by removing the solvent and drying. The isolated material is ground to a fine powder and is pressed by compaction or extrusion into an implant, preferably a cylindrical implant. The compacted material is coated with a biodegradable or non-biodegradable coating by conventional techniques. Prior to implantation a specified area of the coating is removed to expose the compacted drug. For example, the coating may be cut off at one or both of the ends of a cylindrical implant.

It has been shown in an *in vivo* sheep study that the desirable dissolution characteristics of the compositions of the invention are maintained and that the release profiles for *in vivo* release and *in vitro* dissolution are comparable, demonstrating the effectiveness of the present compositions for the parenteral administration of Formula (I) phenylethanolamine derivatives. In a controlled experiment, a compacted 3.2 mm (⅛") cylindrical implant, containing 50% on a weight basis of a Formula (I) phenylethanolamine compound previously described, 45% on a weight basis of castorwax, and 5% on a weight basis of ethylcellulose, and coated with a non-biodegradable coating, released 0.11 mg/day to 1.28 mg/day of said phenylethanolamine compound from the implant *in vivo*, compared to 0.13 mg/day to 0.82 mg/day *in vitro*.

The invention is further illustrated by the following examples.

Examples 1—10

Preparation of Salts of Phenylethanolamine Derivatives

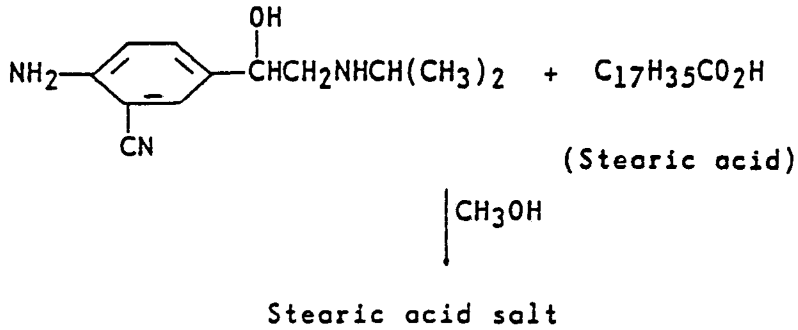


5-[1-Hydroxy-2-(isopropylamino)ethyl] anthranilonitrile (0.7458 g, 0.00262 mol) and stearic acid (1.04 molar equivalents) are added to hot methyl alcohol (6 g) and the resulting solution is stirred at reflux for one hour. The resulting mixture is cooled and the methyl alcohol is removed under reduced pressure. The resulting molten material, which solidified on standing, is ground to a fine powder, and dried in a vacuum, and further dried in a vacuum oven at 55°C. The resulting solid is washed with several aliquots of anhydrous diethyl ether to remove excess stearic acid, and is dried to give 1.016 g, 88.5% yield of the desired salt.

Salts of the phenylethanolamine derivatives are prepared using the above procedure and the appropriate phenylethanolamine and organic acids. Table I contains an element analysis of ten phenylethanolamine derivatives.

TABLE I

Elemental analysis of different phenylethanolamine salts

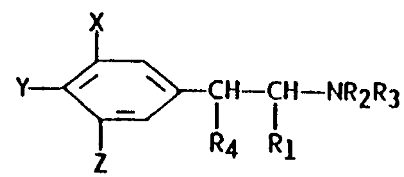

| Example No | $R_1$ | $R_2$ | $R_3$ | $R_4$ | X | Y | Z | Salt type | Found % C | Found % H | Found % N | Calculated % C | Calculated % H | Calculated % N |
|---|---|---|---|---|---|---|---|---|---|---|---|---|---|---|
| 1 | H | H | $i$-$C_3H_7$ | OH | H | $NH_2$ | CN | Stearate | 71.46 | 10.47 | 8.41 | 71.57 | 10.54 | 8.35 |
| 2 | H | H | $i$-$C_3H_7$ | OH | H | $NH_2$ | CN | Caproate | 64.52 | 8.29 | 12.26 | 64.40 | 8.64 | 12.52 |
| 3 | H | H | $i$-$C_3H_7$ | OH | H | $NH_2$ | CN | Benzoate | 64.14 | 6.42 | 11.98 | 66.78 | 6.74 | 12.30 |
| 4 | H | H | $i$-$C_3H_7$ | OH | H | $NH_2$ | CN | Pamoate | 64.66 | 5.92 | 9.07 | 68.20 | 6.05 | 10.16 |
| 5 | H | H | $i$-$C_3H_7$ | OH | H | $NH_2$ | CN | Myristate | 69.95 | 9.81 | 9.06 | 69.69 | 10.05 | 9.38 |
| 6 | H | H | $i$-$C_3H_7$ | OH | H | $NH_2$ | CN | Fumarate | 59.31 | 6.70 | 14.53 | 60.58 | 6.85 | 15.14 |
| 7 | H | H | $t$-$C_4H_9$ | OH | Cl | $NH_2$ | Cl | Myristate | 61.12 | 8.40 | 5.53 | 61.79 | 9.11 | 5.55 |
| 8 | H | H | $t$-$C_4H_9$ | OH | Cl | $NH_2$ | Cl | Stearate | 64.80 | 7.87 | 4.99 | 64.18 | 9.63 | 4.99 |
| 9 | H | H | $t$-$C_4H_9$ | OH | F | H | CN | Myristate | 70.11 | 9.28 | 6.03 | 69.83 | 9.70 | 6.03 |
| 10 | H | H | $t$-$C_4H_9$ | OH | F | H | CN | Stearate | 72.03 | 10.25 | 5.44 | 71.54 | 10.19 | 5.38 |

Example 11

Preparation of the Partially Coated Compacted Implants of Phenylethanolamine Derivatives

Implants are prepared by weighing a sufficient quantity of the ground homogeneous mixture of the desired salt or of pure phenylethanolamine and the desired diluents. The mixture is then compressed on a carver press at from 70—350 bar (1000 to 5000 psig) in a 4.8 mm (3/16'') or 3.2 mm (1/8'') diameter cylindrical die. Smaller implants are prepared by compressing the appropriate quantity of the compound or acid salt on a rotary tablet press using a 1/8'' diameter punch and die to give cylindrical implants.

The implants prepared above are coated with both diodegradable and non-biodegradable coatings by procedures A and B below.

Procedure A

*Non-Biodegradable Silicon Polymer*

Clean grade silicon elastomer (10 parts) is mixed with curing agent (one part) on a watch glass with a spatula. This mixture is deaerated in a dessicator for 30 minutes. The implants are grasped by the ends with tweezers, rolled into the silicon polymer, placed on end on aluminum foil and cured at 40°C overnight. One or both of the ends are removed with a razor blade leaving the "shaft" of the cylinder coated.

Alternatively, implants may be dip coated with 20% to 40% of a medical adhesive, sold under the trademark SILASTIC® by Dow Corning, which has been dispersed in hexane, and dried and cured at 40°C to 50°C overnight before removing the coating from one or both of the base ends.

Procedure B

*Biodegradable Coatings*

The polymer or copolymer (one part) is dissolved in chloroform (three to eight parts). Each implant is grasped by the ends with tweezers, dipped into the polymer solution, and then the chloroform evaporated at room temperature. After the caoting dries overnight at room temperature, the polymer ends are removed with a razor blade, leaving the long cylindrical "shaft" coated.

Table II below contains a summary of the physical data on the coated implants thus prepared.

TABLE II

| Implant No | Example No | % Phenyl ethanolamine as free base | Magnesium stearate % | Ethyl cellulose % | Castor-wax % | Wt (mg) | Length (mm) | Coating | Surface for release (ends open) |
|---|---|---|---|---|---|---|---|---|---|
| 1 | 1 | (Free base) 80 | - | 5.0 | 15 | 58.5 | 6.6 | Non-biodegradable | 1 |
| 2 | 1 | (Free base) 70 | - | 5.0 | 25 | 57.5 | 6.6 | Non-biodegradable | 1 |
| 3 | 1 | (Free base) 60 | - | 5.0 | 35 | 56.9 | 6.4 | Non-biodegradable | 1 |
| 4 | 1 | (Free base) 50 | - | 5.0 | 45 | 56.9 | 6.4 | Non-biodegradable | 1 |
| 5 | 1 | (Free base) 96 | 0.5 | 3.5 | - | 52.0 | ~6.0 | Biodegradable | 2 |
| 6 | 1 | (Free base) 96 | 0.5 | 3.5 | - | 54.0 | ~6.0 | Non-biodegradable | 1 |
| 7 | 1 | (Free base) 96 | 0.5 | 3.5 | - | 54.0 | ~6.0 | Biodegradable | 1 |
| 8 | 1 | (Free base) 96 | 0.5 | 3.5 | - | 52.0 | ~6.0 | Non-biodegradable | 2 |

TABLE II (Continued)

| Implant No | Example No | % Phenyl ethanolamine as free base | Magnesium stearate % | Ethyl cellulose % | Castor-wax % | Wt (mg) | Length (mm) | Coating | Surface for release (ends open) |
|---|---|---|---|---|---|---|---|---|---|
| 9 | 1 | (Stearate) 96 | - | 4.0 | - | 54 | 6.2 | Non-biodegradable | 1 |
| 10 | 1 | (Stearate) 96 | - | 4.0 | - | 54.0 | 6.2 | Non-biodegradable | 2 |
| 11 | 2 | (Caproate) 95 | 1.0 | 3.5 | - | ~59.5 | ~6.0 | Non-biodegradable | 1 |
| 12 | 3 | (Benzoate) 95 | 1.0 | 3.5 | - | 60.4 | ~6.0 | Non-biodegradable | 1 |
| 13 | 4 | (Pamoate) 95 | 1.0 | 3.5 | - | 49.4 | ~6.0 | Non-biodegradable | 1 |
| 14 | 5 | (Myrisate) 95 | 1.0 | 3.5 | - | ~61.4 | ~6.0 | Non-biodegradable | 1 |
| 15 | 6 | (Fumarate) 95 | 1.0 | 3.5 | - | 57.5 | ~6.0 | Non-biodegradable | 1 |

TABLE II (Continued)

| Implant No | Example No | % Phenyl ethanolamine as free base | Magnesium stearate % | Ethyl cellulose % | Castor-wax % | Wt (mg) | Length (mm) | Coating | Surface for release (ends open) |
|---|---|---|---|---|---|---|---|---|---|
| 16 | 7 | (Myristate) 50 | - | 5.0 | 45.0 | 62.5 | 7.0 | Non-biodegradable | 1 |
| 17 | 8 | (Stearate) 50 | - | 5.0 | 45.0 | 64.0 | 7.5 | Non-biodegradable | 1 |
| 18 | 7 | (Free base) 50 | - | 5.0 | 45.0 | 63.0 | - | Non-biodegradable | 1 |
| 19 | 9 | (Myristate) 50 | - | 5.0 | 45.0 | 62.8 | 7.2 | Non-biodegradable | 1 |
| 20 | 10 | (Stearate) 50 | - | 5.0 | 45.0 | 63.4 | 7.4 | Non-biodegradable | 1 |
| 21 | 9 | (Free base) 50 | - | 5.0 | 45.0 | 58.0 | - | Non-biodegradable | 1 |
| 22 | 7 | (Myristate) 95 | 1.0 | 4.0 | - | 63.2 | 7.0 | Non-biodegradable | 1 |

Example 12

Dissolution Experiments

Dissolution experiments are carried out at 390°C using a shaking bottle method. Phosphate buffered saline (PBS) adjusted to pH −7.1 is the dissolution medium ($NaH_2PO_4 \cdot H_2O$ 3.45 g, $Na_2HPO_4$ 3.55 g NaCl 9.50 g dissolved in distilled water to 1000 ml).

The implant is placed in a disposable polypropylene flat base tube (Sarstedt No. 58.537 with No. 67.790 cap) and 30 ml PBS is added. The tubes are placed in a 39°C Fisher Shaking Water Bath (Model 129 — shaker setting at 2¾). The PBS is changed daily, with each measurement and samples are assayed by optical density measurement at 321 nm or 287 nm to give the dissolution profiles.

The results of these experiments are summarized in Tables III, IV, and V below. Table III shows the dissolution of uncoated implants of pure Formula I compounds in an aqueous physiological environment at 39°C from 11 to 17 days. The dissolution kinetics and rate are altered by coating the cylindrical implant and having one or both of the ends exposed. Table IV illustrates the improvement in dissolution profiles of the implants obtained with biodegradable and non-biodegradable coating material. The use of free bases and different $C_{10}$—$C_{20}$ fatty acid salts in conjunction with diluents and various coatings on the compacted implants provides a means of regulating the release rate of the compounds over extended periods of time. Table V represents the variations in the release profiles obtained with various salts of Formula I compounds having the structure

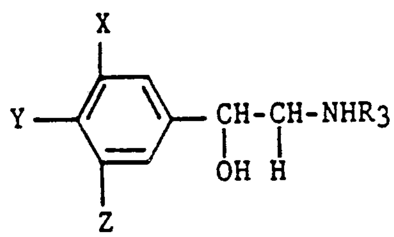

(I)

wherein $R_3$ is *i*-$C_3H_7$ or *t*-$C_4H_9$; X is hydrogen or halogen; Y is hydrogen or $NH_2$; Z is halogen or CN, obtained with a non-biodegradable coating material.

TABLE III

Dissolution of uncoated compacted implants of Formula I Compounds

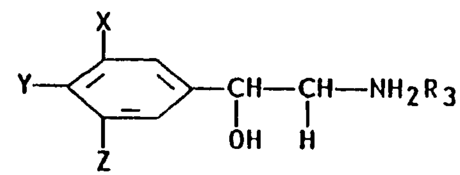

| Compound | | | | | Castor wax | Ethyl-cellulose | Range of release | Days until |
|---|---|---|---|---|---|---|---|---|
| $R_3$ | X | Y | Z | % | % | % | mg/day | exhausted |
| $i$-$C_3H_7$ | H | $NH_2$ | CN | 80 | 15 | 5 | 1.53 - 14.52 | 11 |
| $i$-$C_3H_7$ | H | $NH_2$ | CN | 70 | 25 | 5 | 1.79 - 13.1 | 11 |
| $i$-$C_3H_7$ | H | $NH_2$ | CN | 60 | 35 | 5 | 0.22 - 11.21 | 12 |
| $i$-$C_3H_7$ | H | $NH_2$ | CN | 50 | 45 | 5 | 0.56 - 9.49 | 14 |
| $t$-$C_4H_9$ | Cl | $NH_2$ | Cl | 50 | 45 | 5 | 0.38 - 8.71 | 17 |

TABLE IV

Dissolution of partially coated compacted implants of Formula I compounds

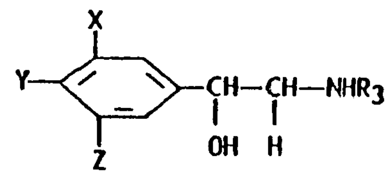

| Compound | | | | | Magnesium Stearate | Castor wax | Ethyl cellulose | Coating | Surface for release (ends open) | Range of release mg/day | Days until exhausted |
|---|---|---|---|---|---|---|---|---|---|---|---|
| $R_3$ | X | Y | Z | % | % | % | % | | | | |
| $i$-$C_3H_7$ | H | $NH_2$ | CN | 80 | - | 15 | 5.0 | Non-biodegradable | 1 | .24 - 5.15 | 43 |
| $i$-$C_3H_7$ | H | $NH_2$ | CN | 70 | - | 25 | 5.0 | Non-biodegradable | 1 | .6 - 1.73 | 39 |
| $i$-$C_3H_7$ | H | $NH_2$ | CN | 60 | - | 35 | 5.0 | Non-biodegradable | 1 | 0.3 - 2.72 | 46 |
| $i$-$C_3H_7$ | H | $NH_2$ | CN | 50 | - | 45 | 5.0 | Non-biodegradable | 1 | 0.13 - 0.82 | 67 |
| $i$-$C_3H_7$ | H | $NH_2$ | CN | 96 | 0.5 | - | 3.5 | Biodegradable | 2 | 1.9 - 3.41 | 22 |

TABLE IV (Continued)

| Compound | | | | | Magnesium Stearate | Castor wax | Ethyl cellulose | | Surface for release | Range of release | Days until |
|---|---|---|---|---|---|---|---|---|---|---|---|
| $R_3$ | X | Y | Z | % | % | % | % | Coating | (ends open) | mg/day | exhausted |
| $i$-$C_3H_7$ | H | $NH_2$ | CN | 96 | 0.5 | - | 3.5 | Non-biodegradable | 1 | 0.57 - 1.69 | 50 |
| $i$-$C_3H_7$ | H | $NH_2$ | CN | 96 | 0.5 | - | 3.5 | Biodegradable | 1 | 0.17 - 4.98 | 27 |
| $i$-$C_3H_7$ | H | $NH_2$ | CN | 96 | 0.5 | - | 3.5 | Non-biodegradable | 2 | 0.48 - 8.17 | 22 |
| $t$-$C_4H_9$ | Cl | $NH_2$ | Cl | 50 | - | 45 | 5.0 | Non-biodegradable | 1 | 0.22 - 0.99 | 52 |
| $t$-$C_4H_9$ | F | H | CN | 50 | - | 45 | 5.0 | Non-biodegradable | 1 | 0.2 - 4.43 | 17 |

**TABLE V**

Dissolution of partially coated compacted implants of salts of Formula I compounds

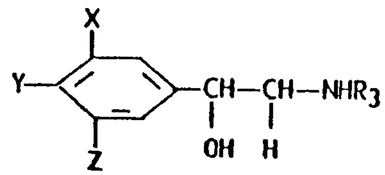

| Salt | Compound R3 | X | Y | Z | % free base | Magnesium stearate % | Ethyl cellulose % | Castor wax % | Surface for release (ends open) | Range of release mg/day |
|---|---|---|---|---|---|---|---|---|---|---|
| Stearate | $i\text{-}C_3H_7$ | H | $NH_2$ | CN | 96 | - | 4.0 | - | 1 | 0.07 - 0.35 |
| Caproate | $i\text{-}C_3H_7$ | H | $NH_2$ | CN | 95 | 1.0 | 4.0 | - | 1 | 1.2 - 29.5 |
| Benzoate | $i\text{-}C_3H_7$ | H | $NH_2$ | CN | 95 | 1.0 | 4.0 | - | 1 | 0.91 - 11.67 |
| Pamoate | $i\text{-}C_3H_7$ | H | $NH_2$ | CN | 95 | 1.0 | 4.0 | - | 1 | 0.4 - 1.87 |
| Myristate | $i\text{-}C_3H_7$ | H | $NH_2$ | CN | 95 | 1.0 | 4.0 | - | 1 | 0.24 - 2.14 |
| Fumarate | $i\text{-}C_3H_7$ | H | $NH_2$ | CN | 95 | 1.0 | 4.0 | - | 1 | 0.59 - 8.97 |
| Myristate | $t\text{-}C_4H_9$ | Cl | $NH_2$ | Cl | 50 | - | 5.0 | 45.0 | 1 | 0.12 - 0.52 |
| Stearate | $t\text{-}C_4H_9$ | Cl | $NH_2$ | Cl | 50 | - | 5.0 | 45.0 | 1 | 0.05 - 0.295 |
| Myristate | $t\text{-}C_4H_9$ | F | H | CN | 50 | - | 5.0 | 45.0 | 1 | 0.056- 0.068 |
| Stearate | $t\text{-}C_4H_9$ | F | H | CN | 50 | - | 5.0 | 45.0 | 1 | 0.012- 0.057 |
| Myristate | $t\text{-}C_4H_9$ | Cl | $NH_2$ | Cl | 95 | 1.0 | 4.0 | - | 1 | 0.17 - 0.57 |

Example 14

Dissolution Characteristics of a Partially Coated Compacted Implant of Carbon 14 labeled 5-[1-hydroxy-2-(isopropylamino)ethyl]anthranilonitrile

I. Preparation of Carbon 14 Labeled Material for Implant Fabrication

Carbon 14 labeled and cold samples of the title phenylethanolamine compound in approximately 1:9 weight ratio are dissolved in methanol and solvent removed under reduced pressure to give a homogeneous sample. Requisite amounts of $C^{14}$-labeled compound, castor wax and ethylcellulose are dissolved in methanol with heating and then the methanol is evaporated off to make to different compositions corresponding to Implant No. 2 (i.e. 70% drug, 25% castor wax, 5% ethylcellulose) and Implant No. 4 (i.e. 50% drug, 45% castor wax, 5% ethylcellulose). The residue is ground to a fine powder.

II. Implant Fabrication

A carver press (Model M) and 3.2 mm ($\frac{1}{8}$'') diameter cylindrical die are used to compress 71.4 mg (Implant No. 4) or 91.8 mg (Implant No. 2) of the preparation from I above 210 bar (3000 psig) for ten minutes to yield a 3.2 mm ($\frac{1}{8}$'') diameter cylindrical implant. Alternatively, the implants are extruded using a laboratory Mini Max Molder (Custom Scientific Instruments) directly into a custom made 3.2 mm ($\frac{1}{8}$'') diameter cylindrical die.

III. Implant Coating

A non-biodegradable polymer coating is applied by grasping each of the implants with tweezers and dipping into a homogeneous coating solution consisting of ten parts by weight silicon elastomer and one part by weight curing agent in 20 parts by weight of hexane. The tipped implants are then dried at room temperature for two hours and then cured at 50—55°C for 12 hours. The polymer coating is removed from one end by slicing, leaving a cylinder with one end surface exposed.

IV. Implant Administration and Release *in vivo*

Two white face crossbread lambs weighing 35—40 kg each housed in metabolism cages and receiving *ad libitum* sheep grower ration No 619 plus hay, and water *ad libitum* are acclimated to cages for four days before implantation. The lambs are implanted each with one implant using an implanting gun.

| Lamb | Implant No. |
|---|---|
| 1A | 2 |
| 1B | 4 |

The total amount of urine from each of the lambs is collected daily and samples analysed for radioactivity with the standard AQUASOL-2 cocktail liquid scintillation fluid using a Beckman Scintillation Counter. At the completion of the experiment, the lambs are sacrificed, the implants removed and examined for physical integrity and extracted with 100 ml of methanol to determine the residual radioactivity in the implants.

The results of these experiments which are summarized in Table VI below demonstrate the effectiveness of the partially coated implant composition of phenylethanolamine derivatives for administration of these compounds *in vivo*. The results of these experiments also demonstrate a uniform release of drug over a period of 30 days.

TABLE VI

In vivo release of $C^{14}$-labeled phenylethanolamine implants in lambs

| Implant Number 2 | | | | Implant Number 4 | | | |
|---|---|---|---|---|---|---|---|
| Day | Amount in urine (mg) | Day | Amount in urine (mg) | Day | Amount in urine (mg) | Day | Amount in urine (mg) |
| 1 | 1.46 | 21 | 1.29 | 1 | 0.54 | 21 | 0.65 |
| 2 | 1.61 | 22 | 1.43 | 2 | 0.94 | 22 | 0.73 |
| 3 | 2.00 | 23 | 1.44 | 3 | 1.28 | 23 | 0.59 |
| 4 | 2.01 | 24 | 1.37 | 4 | 1.17 | 24 | 0.54 |
| 5 | 1.78 | 25 | 1.58 | 5 | 1.18 | 25 | 0.57 |
| 6 | 1.36 | 26 | 1.19 | 6 | 1.15 | 26 | 0.46 |
| 7 | 0.35 | 27 | 1.11 | 7 | 1.08 | 27 | 0.34 |
| 8 | 1.92 | 28 | 1.04 | 8 | 1.20 | 28 | 0.31 |
| 9 | 1.76 | 29 | 1.03 | 9 | 1.10 | 29 | 0.28 |
| 10 | 1.62 | 30 | 0.78 | 10 | 0.96 | 30 | 0.28 |
| 11 | 1.82 | 31 | 0.82 | 11 | 1.12 | 31 | 0.29 |
| 12 | 1.45 | 32 | 0.65 | 12 | 0.91 | 32 | 0.27 |
| 13 | 1.60 | 33 | 0.37 | 13 | 0.94 | 33 | 0.17 |
| 14 | 1.37 | 34 | 0.26 | 14 | 0.82 | 34 | 0.13 |
| 15 | 1.55 | 35 | 0.10 | 15 | 0.87 | 35 | 0.11 |
| 16 | 1.92 | 36 | 0.09 | 16 | 0.99 | 36 | 0.04 |
| 17 | 1.47 | 37 | 0.05 | 17 | 0.77 | 37 | 0.03 |
| 18 | 1.53 | 38 | 0.05 | 18 | 0.82 | 38 | 0.03 |
| 19 | 1.47 | 39 | 0.04 | 19 | 0.78 | 39 | 0.03 |
| 20 | 1.62 | 40 | 0.03 | 20 | 0.83 | 40 | 0.02 |

Example 15

Efficacy of Phenylethanolamine Implants

Six week feed and growth performance trials are conducted using the one end open non-biodegradable coated phenylethanolamine implant number 4, each containing 26,5 mg of the drug. Treatment groups in the study includes a control implant (implants without drug) and lambs implanted with one and two implants. Each group has 5 to 33 lambs weighing 30 to 35 kg each. The implants are placed subcutaneously in the midsection of the back of the right ear, using an implanting gun. The lambs receive sheep grower ration number 619 plus hay and water *ad libitum.* Lambs are weighed periodically and feed intake noted and implants are removed and analyzed for remaining drug.

The results of these experiments are summarized in Table VII, which demonstrates the controlled release of phenylethanolamine obtained with the implants of the invention, and in Table VIII, which demonstrate the improvements in weight gains and feed efficiency obtained by the use of the phenylethanolamine implants of the invention.

TABLE VII
Release of phenylethanolamine from implants *in vivo*

| Period (day) | Average drug released mg/day/implant |
|---|---|
| 0 - 20 | 0.87 |
| 20 - 26 | 0.54 |
| 26 - 33 | 0.477 |
| 33 - 41 | 0.26 |
| 41 - 47 | 0.076 |

TABLE VIII
Effect of implant treatment on weight gain and feed efficiency

| | Control (no drug) | One Implant | Two Implants |
|---|---|---|---|
| Average daily wt. gain in grams | 178.0 | 204.0 | 231.0 |
| Average daily feed intake in kilograms | 1.35 | 1.31 | 1.37 |
| Feed intake per unit of gain | 7.64 | 6.49 | 5.96 |

**Claims**

1. A composition for the parenteral administration of an essentially uniform and continuous amount of a phenylethanolamine derivative having the structure:

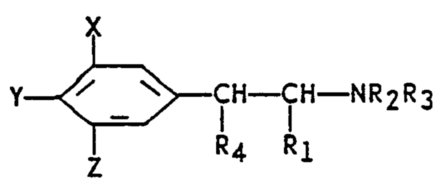

(I)

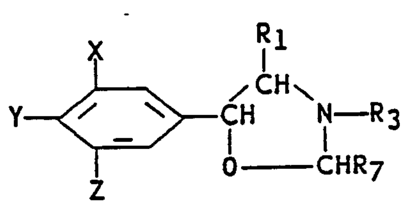

and

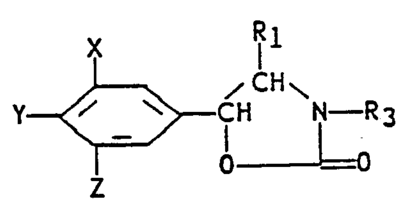

(Ia) (Ib)

wherein,
X is hydrogen, halogen or —CN;
Y is hydrogen, $NR_8R_9$ or $NHCOR_5$;
Z is hydrogen, halogen, OH, CN, $CF_3$, $COOR_1$, $CONH_2$, $C_1$—$C_4$ alkyl, $C_1$—$C_4$ alkoxy, $NO_2$, $C_1$—$C_4$-dialkyl-

aminomethyl or hydroxymethyl;

$R_1$ is hydrogen or $C_1$—$C_4$ alkyl;

$R_2$ is hydrogen, $C_1$—$C_6$ alkyl, $C_3$—$C_4$ alkenyl, $C_2$—$C_5$ alkanoyl or

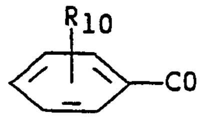 ;

$R_3$ is hydrogen, $C_1$—$C_6$ alkyl, $C_3$—$C_6$ cycloalkyl, methoxypropyl, $C_3$—$C_4$ alkenyl, phenyl, 2-hydroxyethyl, α,α-dimethylphenethyl, benzyl, 3-phenylpropyl or 3-(4-carbomethoxyphenyl)propyl; and when $R_2$ and $R_3$ are taken together with the nitrogen to which they are attached, they represent morpholino or N'-$C_1$—$C_4$ alkylpiperazino;

$R_4$ is hydrogen, OH, $OR_6$ or $SR_{11}$;

$R_5$ is hydrogen, $C_1$—$C_4$ alkyl, $C_1$—$C_4$ alkoxy,

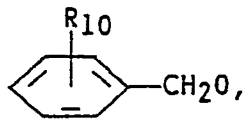, 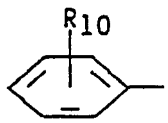 or $N(R_1)_2$ ;

$R_6$ is $C_1$—$C_6$ alkyl, $C_2$—$C_5$ alkanoyl,

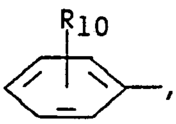, 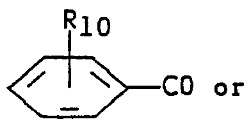 or 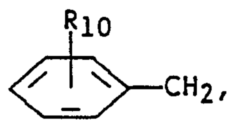, $C_3$–$C_4$ alkenyl;

$R_7$ is hydrogen, $C_1$—$C_4$ alkyl or phenyl;

$R_8$ is hydrogen, $C_1$—$C_4$ alkyl or $C_3$—$C_4$ alkenyl;

$R_9$ is hydrogen, $C_1$—$C_6$ alkyl, $C_4$—$C_6$ cycloalkyl, $C_3$—$C_4$ alkenyl, or benzyl; and when $R_8$ and $R_9$ are taken together with the nitrogen to which they are attached, they represent pyrrolidino;

$R_{10}$ is chloro, dichloro, methyl, dimethyl, methoxy, dimethoxy or nitro; and

$R_{11}$ is $C_1$—$C_6$ alkyl, phenyl or benzyl; and/or is $C_{10}$—$C_{20}$ fatty acid salts, over an extended period of time which comprises a compacted and partially coated phenylethanolamine derivative, which exhibits some degree of solubility in an aqueous physiological environement.

2. A composition according to Claim 1, which further comprises up to 50% by weight of a diluent and up to 5% by weight of a lubricant.

3. A composition according to Claim 1, wherein X is hydrogen or halogen; Y is hydrogen, $NR_8R_9$ or $NHCOR_5$; Z is halogen, OH, CN, $CF_3$, $COOR_1$, $CONH_2$, methyl, methoxy, $NO_2$, $C_1$—$C_4$ dialkylaminomethyl, or hydroxymethyl; wherein X is hydrogen; Y is $NH_2$; Z is CN; $R_3$ is i-$C_3H_7$; $R_1$ and $R_2$ are hydrogen; and $R_4$ is OH; wherein X is Cl; Y is $NH_2$; Z is Cl; $R_3$ is t-$C_4H_9$; and $R_1$ and $R_2$ are hydrogen; and $R_4$ is OH; wherein X is F; Y is hydrogen; Z is CN; $R_3$ is t-$C_4H_9$; $R_1$ and $R_2$ are hydrogen; and $R_4$ is OH; or wherein X and Y are hydrogen; Z is CH; $R_3$ is t-$C_3H_7$; $R_1$ and $R_2$ are hydrogen; and $R_4$ is OH.

4. A composition according to Claim 1, wherein the coating is biodegradable or nonbiodegradable.

5. A method for preparing an implant for the parenteral administration of an essentially uniform and continuous amount of a phenylethanolamine derivative as defined in Claim 1 over an extended period of time which comprises compacting the phenylethanolamine derivative and/or its $C_{10}$—$C_{20}$ fatty acid salt into the form of an implant, coating the implant with a coating material; and removing a section of the coating material from the coated implant to expose the phenylethanolamine derivative.

6. A method according to Claim 5, which further comprises mixing the phenylethanolamine derivative with diluent and/or lubricant prior to the compaction step.

**Patentansprüche**

1. Mittel zur parenteralen Verabreichung einer im wesentlichen einförmigen und kontinuierlichen Menge eines Phenylethanolaminderivats mit der Struktur

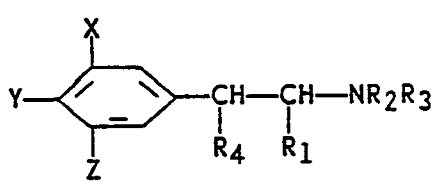

(I)

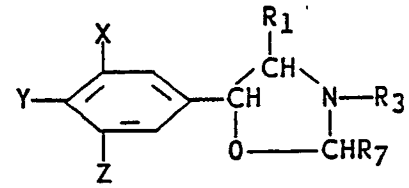 und 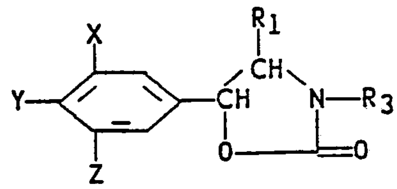

(Ia) (Ib)

über einen längeren Zeitraum, wobei

X für Wasserstoff, Halogen oder —CN steht;

Y für Wasserstoff, $NR_8R_9$ oder $NHCOR_5$ steht;

Z für Wasserstoff, Halogen, OH, CN, $CF_3$, $COOR_1$, $CONH_2$, $C_1$—$C_4$-Alkyl, $C_1$—$C_4$-Alkoxy, $NO_2$, $C_1$—$C_4$-Dialkylaminomethyl oder Hydroxymethyl steht;

$R_1$ für Wasserstoff oder $C_1$—$C_4$-Alkyl steht;

$R_2$ für Wasserstoff, $C_1$—$C_6$-Alkyl, $C_3$—$C_4$-Alkenyl, $C_2$—$C_5$-Alkanoyl oder

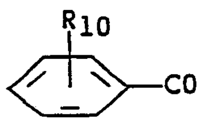 ;

steht:

$R_3$ für Wasserstoff, $C_1$—$C_6$-Alkyl, $C_3$—$C_6$-Cycloalkyl, Methoxypropyl, $C_3$—$C_4$-Alkenyl, Phenyl, 2-Hydroxyethyl, α,α-Dimethylphenethyl, Benzyl, 3-Phenylpropyl oder 3-(4-Carbomethoxyphenyl)propyl steht; und falls $R_2$ und $R_3$ zusammengefaßt sind mit dem Stickstoff, an den sie geknüpft sind, für Morpholino oder N'-$C_1$—$C_4$-Alkyl-piperazino stehen;

$R_4$ für Wasserstoff, OH, $OR_6$ oder $SR_{11}$ steht;

$R_5$ für Wasserstoff, $C_1$—$C_4$-Alkyl, $C_1$—$C_4$-Alkoxy,

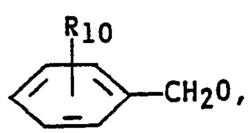 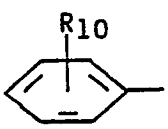 oder $N(R_1)_2$ steht;

$R_6$ für $C_1$—$C_6$-Alkyl, $C_2$—$C_5$-Alkanoyl,

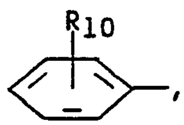 , 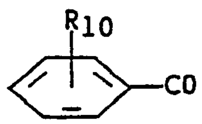 , 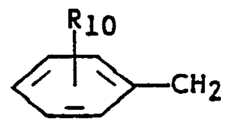 oder $C_3$—$C_4$ Alkenyl steht;

$R_7$ für Wasserstoff, $C_1$—$C_4$-Alkyl oder Phenyl steht;

$R_8$ für Wasserstoff, $C_1$—$C_4$-Alkyl oder $C_3$—$C_4$-Alkenyl steht;

$R_9$ für Wasserstoff, $C_1$—$C_6$-Alkyl, $C_4$—$C_6$-Cycloalkyl, $C_3$—$C_4$-Alkenyl oder Benzyl steht; und falls $R_8$ und $R_9$ zusammengefaßt sind mit dem Stickstoff, an den sie geknüpft sind, für Pyrrolidino stehen;

$R_{10}$ für Chlor, Dichlor, Methyl, Dimethyl, Methoxy, Dimethoxy oder Nitro steht; und

$R_{11}$ für $C_1$—$C_6$-Alkyl, Phenyl oder Benzyl steht; und/oder ihre $C_{10}$—$C_{20}$-Fettsäuresalze, umfassend ein kompaktiertes und teilweise beschichtetes Phenylethanolaminderivat, das in einer wässrigen, physiologischen Umgebung ein gewisses Maß an Löslichkeit zeigt.

2. Mittel gemäß Anspruch 1, ferner umfassend bis zu 50 Gew.-% eines Verdünnungsmittel und bis zu 5 Gew.-% eines Gleitmittels.

3. Mittel gemäß Anspruch 1, wobei X für Wasserstoff oder Halogen steht; Y für Wasserstoff, $NR_8R_9$ oder $NHCOR_5$ steht; Z für Halogen, OH, CN, $CF_3$, $COOR_1$, $COHN_2$, Methyl, Methoxy, $NO_2$, $C_1$—$C_4$-Dialkylaminomethyl oder Hydroxymethyl steht; wobei X für Wasserstoff steht; Y für $NH_2$ steht; Z für CN steht; $R_3$ für i-$C_3H_7$ steht; $R_1$ und $R_2$ Wasserstoff sind; und $R_4$ OH steht; wobei X für Cl steht; Y für $NH_2$ steht; Z für Cl steht; $R_3$ für t-$C_4H_9$ steht; $R_1$ und $R_2$ Wasserstoff sind; und $R_4$ für OH steht; wobei X für F steht; Y für Wasserstoff steht; Z für CN steht; $R_3$ für t-$C_4H_9$ steht; $R_1$ und $R_2$ Wasserstoff sind; und $R_4$ für OH steht; oder wobei X und Y Wasserstoff sind; Z für CN steht; $R_3$ für t-$C_3H_7$ steht; $R_1$ und $R_2$ Wasserstoff sind und $R_4$ für OH steht.

4. Mittel gemäß Anspruch 1, wobei die Beschichtung bioabbaubar oder nicht-bioabbaubar ist.

5. Verfahren zur Herstellung eines Implantats für die parenterale Verabreichung einer im wesentlichen einförmigen und kontinuierlichen Menge eines Phenylethanolaminderivats gemäß Anspruch 1 über einen

längeren Zeitraum, umfassend Kompaktierung des Phenylethanolaminderivats unde/oder seines $C_1$—$C_{20}$-Fettsäuresalzes in die Form eines Implantats, Beschichtung des Implantats mit einem Beschichtungsmaterial; und Entfernung eines Abschnitts des Beschichtungsmaterials von dem beschichteten Implantat, um das Phenylethanolaminderivat freizulegen.

6. Verfahren gemäß Anspruch 5, ferner umfassend das Vermischen des Phenylethanolaminderivats mit Verdünnungsmittel und/oder Gleitmittel vor der Kompaktierungsstufe.

**Revendications**

1. Composition pour l'administration à peu près régulière et continue, par voie parentérale, d'une quantité d'un dérivé de phényléthanolamine, de structure:

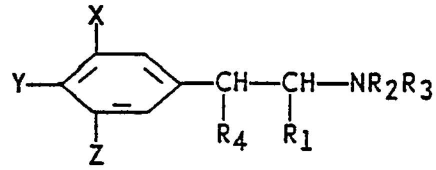

(I)

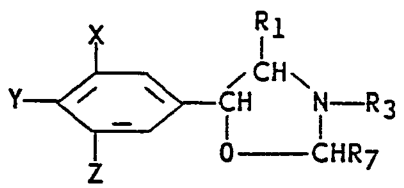

(Ia)

et

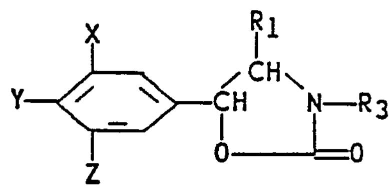

(Ib)

dans laquelle

X représente un atome d'hydrogène, d'halogène ou un groupe —CN;

Y représente un atome d'hydrogène, un groupe $NR_8R_9$ ou $NHCOR_5$;

Z représente un atome d'hydrogène, d'halogène, un groupe OH, CN, $CF_3$, $COOR_1$, $CONH_2$, alkyle en $C_1$—$C_4$, alkoxy en $C_1$—$C_4$, $NO_2$, di(alkyl en $C_1$—$C_4$)aminométhyle ou hydroxyméthyle;

$R_1$ représente un atome d'hydrogène ou un groupe alkyle en $C_1$—$C_4$;

$R_2$ représente un atome d'hydrogène, un groupe alkyle en $C_1$—$C_6$, alkényle en $C_3$—$C_4$, alcanoyle en $C_2$—$C_5$, ou:

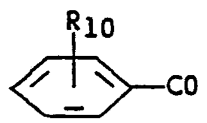 ;

$R_3$ représente un atome d'hydrogène, un groupe alkyle en $C_1$—$C_6$, cycloalkyle en $C_3$—$C_6$, méthoxypropyle, alkényle en $C_3$—$C_4$, phényle, 2-hydroxyéthyle, α,α-diméthylphénéthyle, benzyle, 3-phénylpropyle ou 3-(4-carbométhoxylphényl)propyle; et lorsque $R_2$ et $R_3$ forment un cycle avec l'atome d'azote auquel ils sont liés, ils représentent un groupe morpholino ou N'-(alkyle en $C_1$—$C_4$)pipérazino;

$R_4$ représente un atome d'hydrogène, un groupe OH, $OR_6$ ou $SR_{11}$;

$N_5$ représente un atome d'hydrogène, un groupe alkyle en $C_1$—$C_4$, alkoxy en $C_1$—$C_4$,

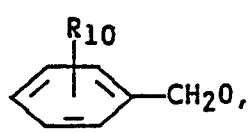, 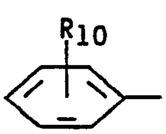 ou $N(R_1)_2$;

$R_6$ représente un groupe alkyle en $C_1$—$C_6$, alcanoyle en $C_2$—$C_5$,

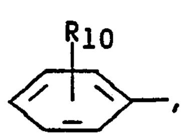, 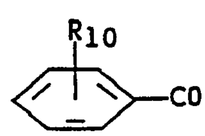, 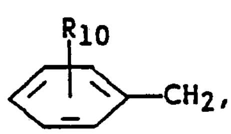, ou alkényle en $C_3$—$C_4$;

$R_7$ représente un atome d'hydrogène, un groupe alkyle en $C_1$—$C_4$ ou phényle;

$R_8$ représente un atome d'hydrogène, un groupe alkyle en $C_1$—$C_4$ ou alkényle en $C_3$—$C_4$;

$R_9$ représente un atome d'hydrogène, un groupe alkyle en $C_1$—$C_6$, cycloalkyle en $C_4$—$C_6$, alkényle en $C_3$—$C_4$ ou benzyle; et lorsque $R_8$ et $R_9$ forment ensemble un cycle avec l'atome d'azote auquel ils sont liés, ils représentent un groupe pyrrolidino;

$R_{10}$ représente un groupe chloro, dichloro, méthyle, diméthyle, méthoxy, diméthoxy ou nitro; et

$R_{11}$ représente un groupe alkyle en $C_1$—$C_6$, phényle ou benzyle;

et/ou ses sels avec un acide gras en $C_{10}$ à $C_{20}$, pendant une période prolongée, comprenant un dérivé de phényléthanolamine compacté et partiellement enrobé, ayant un certain degré de solubilité dans un milieu physiologique aqueux.

2. Composition selon la revendication 1, comprenant en outre jusqu'à 50% en poids d'un diluant, et jusqu'à 5% en poids d'un lubrifiant.

3. Composition selon la revendication 1, dans laquelle X représente un atome d'hydrogène ou d'halogène; Y représente un atome d'hydrogène, un groupe $NR_8R_9$ ou $NHCOR_5$; Z représente un atome d'halogène, un groupe OH, CN, $CF_3$, $COOR_1$, $CONH_2$, méthyle, méthoxy, $NO_2$, di(alkyl en $C_1$—$C_4$)amino-méthyle ou hydroxyméthyle; dans laquelle X représente un atome d'hydrogène; Y représente un groupe $NH_2$; Z représente un groupe CN; $R_3$ représente un groupe iso-$C_3H_7$; $R_1$ et $R_2$ représentent des atomes d'hydrogène; et $R_4$ représente un groupe OH; dans laquelle X représente un atome de chlore; Y représente un groupe $NH_2$; Z represénte un atome de chlore; $R_2$ représente un groupe tert-$C_4H_9$; $R_1$ et $R_2$ représentent des atomes d'hydrogène; et $R_4$ représente un groupe OH; dans laquelle X représente un atome de fluor; Y représente un atome d'hydrogène; Z représente un groupe CN; $R_3$ représente un groupe tert-$C_4H_9$; $R_1$ et $R_2$ représentent des atomes d'hydrogène; et $R_4$ représente un groupe OH; ou dans laquelle X et Y représentent des atomes d'hydrogène; Z représente un groupe CN; $R_3$ représente un groupe tert-$C_3H_7$; $R_1$ et $R_2$ représente des atomes d'hydrogène; et $R_4$ représente un groupe OH.

4. Composition selon la revendication 1, dans laquelle l'enrobage est biodégradable ou non biodégradable.

5. Procédé de préparation d'un implant pour l'administration presque régulière et continue, par voie parentérale, d'un dérivé de phényléthanolamine selon la revendication 1, pendant une période prolongée, dans lequel on compacte le dérivé de phényléthanolamine et/ou son sel avec un acide gras en $C_{10}$ à $C_{20}$, sous la forme d'un implant, on revêt l'implant avec un matériau d'enrobage; et on élimine une partie du matériau d'enrobage de l'implant enrobé, afin d'exposer le dérivé de phényléthanolamine.

6. Procédé selon la revendication 5, dans lequel on mélange en outre le dérivé de phényléthanolamine avec un diluant et/ou un lubrifiant, avant l'opération de compactage.